# EUROPÄISCHE PATENTSCHRIFT

(11) **EP 1 204 407 B1**
(45) Veröffentlichungstag und Bekanntmachung des Hinweises auf die Patenterteilung: **27.10.2004**
(21) Anmeldenummer: 00963870.1
(22) Anmeldetag: 04.08.2000
(51) Int. Cl.: A61K 9/127

(54) **IMPLANTIERBARES WIRKSTOFFDEPOT**
IMPLANTABLE ACTIVE INGREDIENT DEPOT
DEPOT DE PRINCIPE ACTIF IMPLANTABLE

(30) Priorität: 06.08.1999 DE 19938331
(43) Veröffentlichungstag der Anmeldung: 15.05.2002
(73) Patentinhaber: MAX-DELBRÜCK-CENTRUM FÜR MOLEKULARE MEDIZIN, 13125 Berlin (DE)
(72) Erfinder: RESZKA, Regina, 16341 Schwanebeck (DE); SCHLÜTER, Roland, 59494 Soest (DE)
(74) Vertreter: Baumbach, Friedrich, Dr.
(86) Internationale Anmeldenummer: PCT/DE2000/002615
(87) Internationale Veröffentlichungsnummer: WO 2001/010411

(56) Entgegenhaltungen:
- WO-A-93/19737
- WO-A-96/39125
- WO-A-98/47487
- DE-A- 4 430 593
- DE-A- 19 724 796
- US-A- 5 891 456

## Beschreibung

Die Erfindung betrifft ein implantierbares Wirkstoffdepot für therapeutisch wirksame Substanzen. Anwendungsgebiete sind die Medizin und die pharmazeutische Industrie.

Ein Ziel der pharmazeutischen Forschung besteht darin, die Wirkstoffzufuhr an den Patienten möglichst kontinuierlich zu gestalten. So ist es eine große Erleichterung für Diabeteskranke, anstelle der mehrmals am Tage durchzuführenden Injektionen Depotinsulin als Therapeutikum zur Verfügung zu haben. Neuerdings werden auch Depot-Zytostatika (Polymer-gebunden) als intratumorale Freisetzungssysteme eingesetzt. (Walter et al. Neurosurgery 37 (6) 1995 (Review) "Intratumorale Chemotherapie").

In EP 126 751 werden Präparationen beschrieben, die eine Mischung eines biologisch wirksamen Materials und einer oder mehrerer amphiphiler Substanzen enthält, wobei diese Substanzen zusammen mit Flüssigkeiten zur Bildung einer flüssigen kristallinen Phase befähigt sein müssen. Die wichtigste amphiphile Substanz ist Monoolein, wobei die thermotropen und lyotropen Mesophasen mit Wasser im Vordergrund stehen. Das Ziel der Präparationen besteht darin, die langsame und gleichmäßige Freisetzung des biologisch wirksamen Materials (z. B. Benzylpenicillin, Insulin) am Wirkungsort zu erreichen und sie vor störenden Wechselwirkungen mit dem Organismus zu schützen.

Die in EP 126 751 angegebenen Präparationen sind für eine systemische Anwendung entwickelt worden. Das geht auch aus späteren Veröffentlichungen der Autoren hervor, wobei die Zielrichtung darin bestand, die Größe der kubischen Phase auf eine für die systemische Anwendung geeignete Größe (< 10 µm) zu verringern (S. Engström, Lipid Technology, Vol. 2, No. 2, April 1990, S. 42-45).

Des weiteren sind die Präparationen nur für eine antibiotische Anwendung entwickelt worden.

In WO-A-9639125 werden Zusanmensetzungen und Methoden zur Verabreichung von Biokatalysatoren (Enzymen) unter Verwendung einer hoch viskosen Kubischen Phase als Matrix beschrieben.

In WO-A-9847487 wird die Verabreichung einer Kubischen Lipid-Wasser-Phase zur Wirkstoffreisetzung beschrieben.

Ziel der Erfindung ist der Einsatz der gelartigen kubischen Mesophasen des Systems Monoolein-Wasser als implantierbares Wirkstoffdepot zur Behandlung von Tumoren in der Krebstherapie und in der Gentherapie. Es soll ein rationales Membrandesign entwickelt werden, um die Freigabe der Wirkstoffe hinsichtlich des Zeitraums und der Menge steuern zu können.

Dieses Ziel wird mit den in den Ansprüchen angegebenen Maßnahmen erreicht.

Ein wesentlicher Vorteil dieser Erfindung besteht darin, daß das Wirkstoff depot vollständig biologisch abbaubar ist. Es kann auf offen liegende Gewebe aufgebracht werden (z. B. nach Operationen) und haftet überraschenderweise sehr gut an Schleimhäuten. Damit ist eine effektive lokoregionale Behandlung von Tumoren und die Beseitigung restenotischer Bereiche möglich.

Ein weiterer Vorteil besteht darin, daß die Freigabe der pharmazeutisch wirksamen Substanzen je nach Wahl des Modifiers hinsichtlich des Zeitraums und der Menge gesteuert werden kann.

Bei Verwendung von polyethylenglykol-modifizierten Lipiden als Bestandteil des Wikstoffdepots kann im Gegensatz zu den unmodifizierten Lipiden eine entscheidende Verlängerung der Abgabe des Wirkstoffs erreicht werden. In Abhängigkeit von der Länge der Polyethylenkette kann eine Feinsteuerung der Freigabe erfolgen. Beispielsweise wird mit einer Länge der Polyethylenkette von 500 Einheiten eine Abgabe des Wirkstoffs über 4 Tage erreicht. Hat die Polyethylenkette eine Länge von 2000 Einheiten, dann verlängert sich Abgabe des Wirkstoffs auf über 7 Tage.

Himtumore sind kaum durch eine systemische Chemotherapie behandelbar, da die meisten Substanzen nicht Bluthirnschrankengängig sind. Eine lokale nach chirurgische Entfernung der Tumorhauptmasse, in Form der Gele eingebrachten Mono- bzw. Polychemotherapie (Carboplatin. und Taxol) verbessert die Aussichten auf eine Lebenszeitverlängerung bei gleichzeitigem Erhalt der Lebensqualität.

Des weiteren läßt sich das erfindungsgemäße Wirkstoffdepot als Doppel-Release-System für Zytostatika zur direkten (lokalen) Chemotherapie verwenden.

Die Erfindung soll nachfolgend durch Ausführungsbeispiele näher erläutert werden.

### Beispiel 1.1 Messung der Freisetzungsraten

Die Bestimmung von Menge und Geschwindigkeit, in der eine eingeschlossene Wirksubstanz aus einer Depotform freigesetzt wird, ist entscheidend für den späteren *in vitro-* und *in vivo*-Einsatz dieses Systems. Es muss versucht werden, eine optimale Form der Freisetzung zu entwickeln, die es gewährleistet, dass das Pharmakon über einen bestimmten Zeitraum in ausreichender Menge - abhängig von der Art des Pharmakons - als Therapeutikum im Körper zur Verfügung steht. Dabei muss die systemische Belastung so niedrig wie möglich gehalten werden, um negative Begleiterscheinungen für das gesunde Gewebe zu vermeiden.

Aus diesem Grund wurden Untersuchungen zur Freisetzungskinetik des slow-Release-Systems durchgeführt.

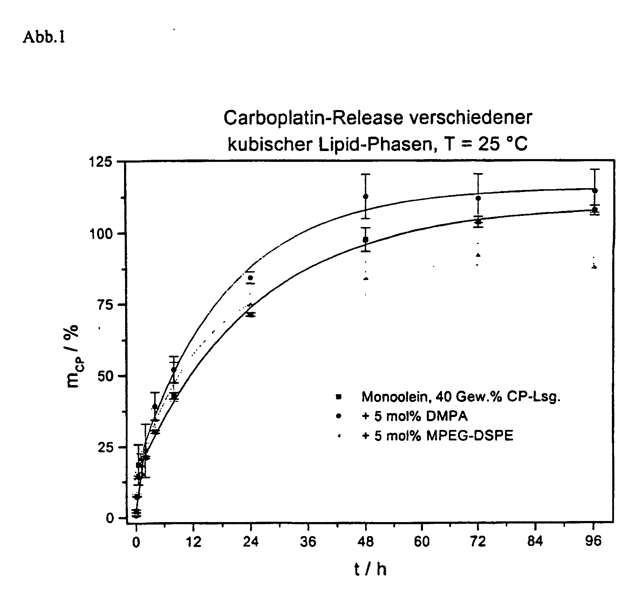

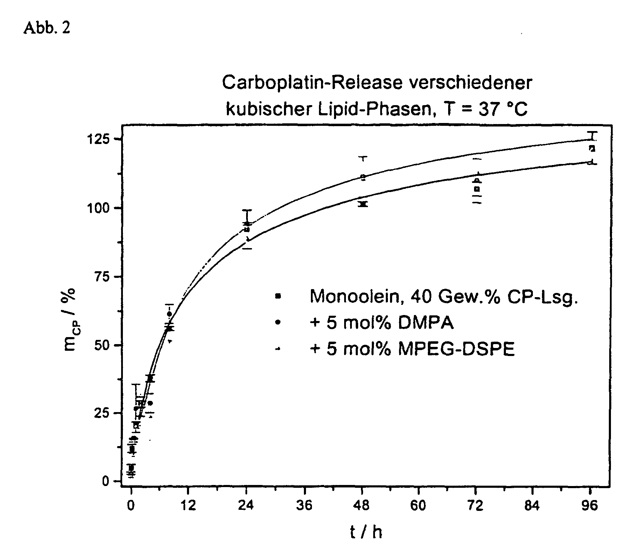

Die Messung der Freisetzungskinetik wurde an kubischen Lipidsystemen und daraus implantierbaren Wirkstoffdepots durchgeführt. Die kubischen Phasen existieren in Excesswasser und sind so relativ stabil gegenüber dem Kontakt mit Körperflüssigkeiten, wie etwa Blut oder Lymphflüssigkeit. Außerdem macht die hohe Viskosität sie recht leicht handhabbar und die Systeme weisen eine gute Haftung an Schleimhäuten und anderem biologischem Gewebe auf, z. B. Beschichtung von Netzen. Das System von dreidimensionalen Wasserkanälen im Inneren der kubischen Phasen führt dazu, dass eine wasserlösliche Substanz wie Carboplatin in den Wasserkanälen inkorporiert wird. Es ist so vor direktem Kontakt mit Körperflüssigkeiten und damit vor Angriffen durch Makrophagen oder Enzymen geschützt und kann so relativ langsam durch die Kanäle hindurch aus dem Depot hinausdiffündieren, wo es dann als wirksame Substanz vorliegt. Wichtige Voraussetzungen sind daher, dass das wasserlösliche Pharmakon möglichst wenig mit der Lipidmembran wechselwirkt und nicht in seiner Struktur - und somit seiner Wirksamkeit - durch chemische Reaktionen verändert wird. Entsprechende physikalisch-chemische Messungen belegen, dass diese Bedingungen von Carboplatin und den kubischen Lipidphasen aus Monoolein, bzw. Monoolein und MPEG-DSPE erfüllt werden (Abb. 1 und 2).

Zum anderen können lipidlösliche Wirkstoffe, wie z. B. Taxol, in die Lipidphase inkorporiert werden. Dadurch ist sowohl ein Ein- wie ein Mehrkomponenten-Freisetzungssystem möglich (Kombinationstherapie)

Nimmt man an, dass die Konzentration an Carboplatin im Inneren der kubischen Phase überall konstant ist, so hängt letztendlich die Diffusion aus der kubischen Phase von der Größe der Grenzfläche zwischen der Probe und dem umgebenden Medium ab und von dem Volumen der Phase an sich, die bei - angenommen - konstanter Oberfläche, die Menge an inkorporiertem Carboplatin festlegt. Oberfläche und Volumen der Probe sind also ausschlaggebende geometrische Faktoren, die die Freisetzungsgeschwindigkeit beeinflussen. Für ein Modellsystem zur Messung der Release-Rate sind diese Parameter also schon möglichst konstant zu wählen. Aus diesem Grunde wurden Probenbehälter mit definiertem Volumen und definierter Grenzfläche verwendet.

### Beispiel 1.1.1 Probenvorbereitung

27 mM Carboplatin (entspricht 10 mg / ml bidest. Wasser) wurden in bidestilliertem Wasser gelöst. Anschließend wurden 5 g Monoolein in ein Gefäß gegeben und im Wasserbad bei ca. 45 °C aufgeschmolzen. Zu der Schmelze wurden 40 Gew.% der CP-Lösung zugesetzt und mit einem Spatel untergerührt. Diese Prozedur wurde 3 mal wiederholt, so dass sich eine homogene kubische Phase ausbilden konnte. Die verschlossenen Behälter wurden 24h auf 40° C temperiert, um eine schnellere Equilibrierung zu erreichen.

Die Systeme mit einem Anteil an MPEG-DSPE oder DMPA wurden analog präpariert - allerdings wurde hier zu der aufgeschmolzenen Monooleinmenge 5 mol% MPEG-DSPE bzw. DMPA zugegeben. Das pulverförmige Zusatzlipid wurde durch heftiges Schütteln im flüssigen MO in Lösung gebracht. Es wurden anschließend wieder 40 Gew.% CP-Lösung zugesetzt und die Probe homogenisiert, wie oben beschrieben.

### Beispiel 1.1.2 Beschreibung des Modellsystems

In ein zylindrisches Probengefäß wurden 236 ± 3 mg kubische Phase eingefüllt. Das entspricht einem Carboplatingehalt von 8,2 ± 0,1 mg pro Gefäß. Die gefüllten Probenbehälter wurden mit ihrer Öffnung in ein temperierbares Volumen von 4 ml bidestilliertem Wasser gehängt, wobei die Kontaktfläche zwischen der kubischen Phase und dem umgebenden Medium exakt 56,7 mm² für jedes Probengefäß betrug. Es wurden jeweils drei Messungen bei 25 und 37 °C unter Schütteln der Probe durchgeführt. In definierten Zeitabständen wurde eine kleine Menge des Überstandes (50 µl) abgenommen und mittels HPLC auf seinen Carboplatingehalt hin untersucht.

Zur Carboplatinbestimmung wurde die sog. Umkehrphasen-HPLC verwendet. Als mobile Phase kam Acetonitril mit 0,015 %iger Phosphorsäure in einem Verhältnis von 89:11 (v/v) zum Einsatz. Die Trennung wurde über eine 25 cm lange MERCK LiChroCart 250-4 Säule (MERCK, Darmstadt) mit einer Partikelgröße von 5 µm erreicht und das Carboplatin mittels UV-Detektion bei 229 nm und einer Durchflussrate der mobilen Phase von 1 ml / min bestimmt (Abb. 1 und 2).

### Beispiel 1.2 Messung der antineoplastischen Wirksamkeit in vitro

Im folgenden Schritt werden Untersuchungen durchgeführt, die die Wirkung einer derartigen Depotform auf lebende Systeme zeigt. Es wurden Tumorzellen F98 verwendet, die gegen Carboplatin sensitiv sind. Zum Einsatz kamen hier Zelllinien eines Ratten-Glioblastoms, die sog. F98-Zelllinie und eines Ratten Kolon-Karzinoms CC531.

### Beispiel 1.2.1 Probenvorbereitung

Die Probenvorbereitung ist identisch mit der in Beispiel 1.1.1 beschriebenen Vorgehensweise. Es wurden Proben präpariert, die unterschiedliche Carboplatinkonzentrationen enthielten. ( 0, 5, 10, 20 und 40 µg Carboplatin je 300 mg kubischer Phase). Im Vergleich zur Messung der modellhaften Freisetzungskinetik wurden sehr geringe Carboplatinkonzentrationen eingesetzt, da die biologischen Systeme extrem empfindlich auf das Zytostatikum reagieren.

### 1.2.2 Beschreibung des Modellsystems

Jeweils 1 ml (5 x 10⁶ Zellen) der einzelnen Zellsuspensionen wurden in eine 24-Well Mikrotiterplatte eingebracht und spezielle Transwell® Kammereinsätze (COSTAR, Niederlande) in die einzelnen Kammern der Mikrotiterplatte eingehängt. Die Einsätze wurden mit jeweils 308 ± 7 mg kubischer Phase (Diffusionsfläche von 33,2 mm²). Die Inkubation der Mikrotiterplatten erfolgte 72 h lang bei 37 °C und 5 Vol.-% CO₂-Zusatz zur Luft. Nach den 72 h wurde die Zellvitalität mittels eines Saure Phosphatase Assays ermittelt.

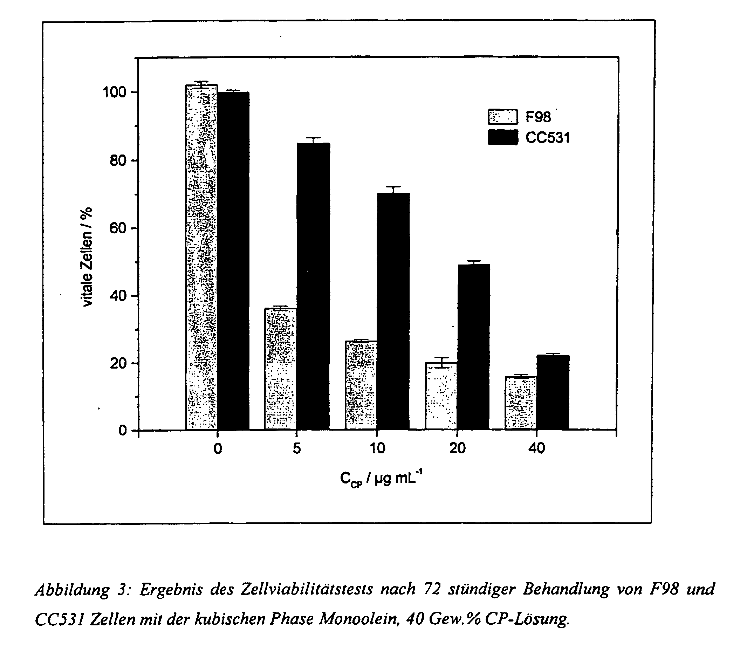

Abbildung 3 zeigt, dass das Freisetzungssystem Monoolein, 40 Gew.% Carboplatinlösung eine Zytotoxizität auf die unterschiedlichen Tumorzelllinien F98 und CC531 hat. Die Kolonkarzimom-Zellen reagieren offensichtlich weitaus weniger sensitiv auf Carboplatin. Hier zeigte sich, dass eine sehr viel höhere Menge Carboplatin (5 µg Carboplatin hat nach 72 h etwa 65 % der Zellen abgetötet) notwendig ist, um den gleichen zytotoxischen Effekt wie bei den Glioblastomzellen zu erreichen. Um ca. 65 % der Zellen zu eliminieren, sind etwa 30 bis 35 µg CP notwendig.

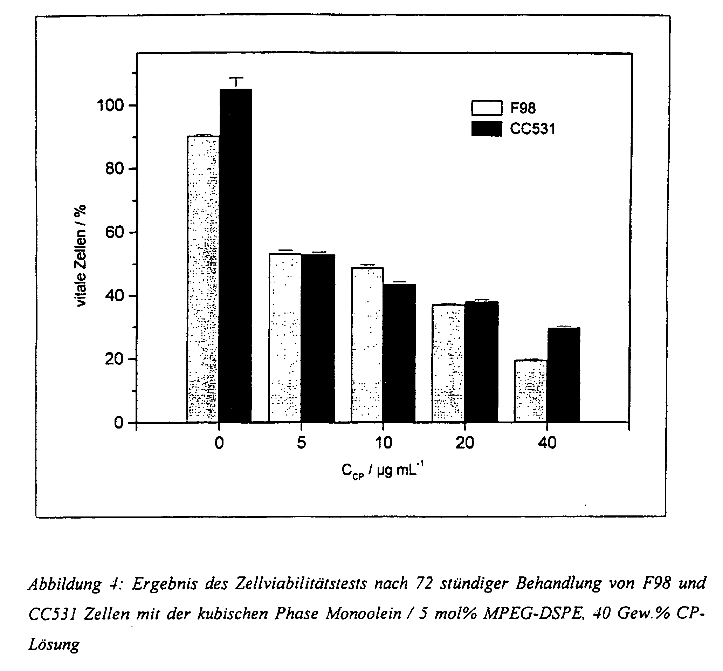

Es ist in Abbildung 4 deutlich der Effekt des freigesetzten Carboplatins auf die Tumorzelllinien zu erkennen. Bei der F98 Zelle ist der Effekt vergleichbar mit dem reinen kubischen System. Bereits eine Menge von 5 µg CP bewirkt eine Zelleliminierung von etwa 65 % nach 72 Stunden. Die Kolonkarzinom-Zelle reagiert aber offensichtlich empfindlicher auf das modifizierte Freisetzungssystem. Es genügen ebenfalls 5 µg Carboplatin, um 65 % der Zellen zu töten.

Vergleicht man nun beide Freisetzungssysteme miteinander, so kommt man zu der Erkenntnis, dass die jeweils unbeladenen kubischen Phasen eine unbedeutende bis nicht messbare Toxizität aufweisen. Mit einer recht geringen Menge von 5 µg Carboplatin lassen sich schon nach 72 Stunden weit über die Hälfte der Zellen im *in vitro*-Versuch abtöten.

## Patentansprüche

1. Implantierbares Wirkstoffdepot, bestehend aus
- einer Lipidmatrix aus Glycerin-Mono-Olein, die in der Lage ist, kubische Phasen zu bilden, in welche
- Lipidmoleküle mit geladenen und/oder sterisch raumfordernden Kopfgruppen als Modifier-Moleküle eingebaut sind, wobei die Modifier-Moleküle die negativ geladene Kopfgruppe 1,2-Dimyristoyl-glycerophosphatidylsäure (DMPA, Na-Salz) tragen oder als Modifier-Moleküle amphiphile Moleküle mit PEG-Kopfgruppen eingesetzt werden, und die
- pharmazeutisch wirksame Substanzen Carboplatin, Oxaliplatin, Taxol, Daunorubicin, Mitoxantron, Gencitabin, Topotecan, Campotecin, Peptide, gentherapeutische Mittel wie DNA, RNA, Oligonukleotide oder Ribozyme enthält.

2. Implantierbares Wirkstoffdepot nach Anspruch 1, wobei die Modifier-Moleküle amphiphile Moleküle mit PEG-Kopfgruppen unterschiedlicher Länge, bevorzugt zwischen 500 und 2000 Einheiten, sind.

3. Implantierbares Wirkstoff depot nach Anspruch 1 und 2, wobei die Modifier-Moleküle 1,2-Distearoyl-glycerophosphatidyl-ethanolamin-methyl-polyethylenglykol sind (MPEG-DSPE).

4. Implantierbares Wirkstoff depot nach Anspruch 1, wobei mehrere pharmazeutisch wirksame Substanzen wie die Kombination von Carboplatin und Taxol eingesetzt werden.

5. Verfahren zur Herstellung eines implantierbaren Wirkstoffdepots gemäß einem der Ansprüche 1 bis 4, **dadurch gekennzeichnet, daß** man der Lipidmatrix, bevorzugt Monoolein, Modifiermoleküle und lipidlösliche pharmazeutisch wirksame Substanzen gemeinsam zusetzt.

6. Verfahren zur Herstellung eines implantierbaren Wirkstoffdepots gemäß einem der Ansprüche 1 bis 4, **dadurch gekennzeichnet, daß** man der Lipidmatrix, bevorzugt Monoolein, Modifiermoleküle zusetzt und nachfolgend mit einer wäßrigen Phase, die die wasserlöslichen pharmazeutisch wirksamen Substanzen enthält, vermischt.

7. Verfahren zur Herstellung eines implantierbaren Wirkstoffdepots gemäß einem der Ansprüche 1 bis 4, **dadurch gekennzeichnet, daß** man der Lipidmatrix, bevorzugt Monoolein, Modifiermoleküle und lipidlösliche pharmazeutisch wirksame Substanzen gemeinsam zusetzt und nachfolgend mit einer wäßrigen Phase, die die wasserlöslichen pharmazeutisch wirksamen Substanzen enthält, vermischt.

8. Verwendung eines implantierbaren Wirkstoffdepots gemäß einem der Ansprüche 1 bis 4 zur Herstellung eines Arzneimittels für die lokale Chemotherapie bzw. Gentherapie von Tumorerkrankungen (u. a. Glioblastome, Hirnmetastasen, Peritonealkarcinose, Blasenkarzinome, Mammarezidive).

9. Verwendung eines implantierbaren Wirkstoffdepots gemäß einem der Ansprüche 1 bis 4 zur Herstellung eines Arzneimittels für die Behandlung arteriosklerotischer Gefäßwände (u. a. Restenosen).

10. Verwendung eines implantierbaren Wirkstoffdepots gemäß einem der Ansprüche 1 bis 4 zur Herstellung eines Arzneimittels für die Behandlung von Parkinson, Alzheimer und multipler Sklerose.

11. Verwendung eines implantierbaren Wirkstoffdepots gemäß einem der Ansprüche 1 bis 4 zur Herstellung eines Arzneimittels, das als slow release System für Schmerztherapeutika (u. a. Morphine) eingesetzt wird.

12. Verwendung eines implantierbaren Wirkstoffdepots gemäß einem der Ansprüche 1 bis 4 zur Herstellung eines Arzneimittels für die Behandlung von rheumatischen Erkrankungen (u. a. rheumatische Arthritis).

13. Verwendung eines implantierbaren Wirkstoffdepots gemäß einem der Ansprüche 1 bis 4 zur Herstellung eines Arzneimittels für die Freisetzung von entzündungshemmenden Substanzen

14. Verwendung eines implantierbaren Wirkstoffdepots gemäß einem der Ansprüche 1 bis 4 zur Herstellung eines Arzneimittels, **dadurch gekennzeichnet, daß** das Wirkstoffdepot auf biologisch abbaubare Netze aufgebracht wird.

15. Verwendung eines implantierbaren Wirkstoffdepots gemäß einem der Ansprüche 1 bis 4 zur Herstellung eines Arzneimittels, welches antiangiogenetische Substanzen und diese Systeme beeinflussende genetische Materialien freisetzt.

16. Verwendung eines implantierbaren Wirkstoffdepots gemäß einem der Ansprüche 1 bis 4 zur Herstellung eines Arzneimittels, welches angiogenetische Substanzen und diese Systeme beeinflussende Materialien freisetzt.

## Claims

1. Implantable active agent depot, comprising
- a lipid matrix of glycerine mono-oleine, which is in a position to form cubic phases, into which
- lipid molecules with charged and/or sterically space-occupying head groups have been installed as modifier molecules, with the modifier molecules bearing the negatively charged head group 1,2-dimyristoyl-glycero-phosphatidic acid (DMPA, Na salt), or amphiphilic molecules with PEG head groups are used as modifier molecules and which
- contains pharmaceutically effective substances such as Carboplatin, Oxaliplatin, Taxol, Daunorubicin, Mitoxantron, Gencitabin, Topotecan, Campotecin, peptides or gene therapeutic agents such as DNA, RNA, oligonucleotides or ribozymes.

2. Implantable active agent depot according to Claim 1, wherein the modifier molecules are amphiphilic molecules with PEG head groups of differing lengths, preferably between 500 and 2000 units.

3. Implantable active agent depot according to Claim 1 and 2, wherein the modifier molecules are 1,2-distearoyl-glycerophosphatidylethanolamine-methyl-polyethylene glycol (MPEG-DSPE).

4. Implantable active agent depot according to Claim 1, wherein a plurality of pharmaceutically effective substances such as a combination of Carboplatin and Taxol are used.

5. Method for the production of an implantable active agent depot according to one of the Claims 1 to 4, wherein modifier molecules and lipid-soluble, pharmaceutically effective substances are jointly added to the lipid matrix, preferably mono-oleine.

6. Method for the production of an implantable active agent depot according to one of the Claims 1 to 4, wherein modifier molecules are added to the lipid matrix, preferably mono-oleine, and are subsequently mixed with a watery phase containing the water-soluble, pharmaceutically effective substances.

7. Method for the production of an implantable active agent depot according to one of the Claims 1 to 4, wherein modifier molecules and lipid-soluble, pharmaceutically effective substances are jointly added to the lipid matrix, preferably mono-oleine, and are subsequently mixed with a watery phase containing the water-soluble, pharmaceutically effective substances.

8. Use of an implantable active agent depot according to one of the Claims 1 to 4 for the production of a drug for local chemotherapy or gene therapy of tumour diseases (inter alia glioblastoma, cranial metastases, peritoneal carcinosis, bladder carcinoma, mamma relapses).

9. Use of an implantable active agent depot according to one of the Claims 1 to 4 for the production of a drug for the treatment of arteriosclerotic vessel walls (inter alia restenoses).

10. Use of an implantable active agent depot according to one of the Claims 1 to 4 for the production of a drug for the treatment of Parkinson, Alzheimer and multiple sclerosis.

11. Use of an implantable active agent depot according to one of the Claims 1 to 4 for the production of a drug used as a slow release system for pain therapeutics (inter alia morphine).

12. Use of an implantable active agent depot according to one of the Claims 1 to 4 for the production of a drug for the treatment of rheumatic diseases (inter alia rheumatic arthritis).

13. Use of an implantable active agent depot according to one of the Claims 1 to 4 for the production of a drug for the release of antiphlogistic substances.

14. Use of an implantable active agent depot according to one of the Claims 1 to 4 for the production of a drug, wherein the active agent depot is attached to biologically decomposable nets.

15. Use of an implantable active agent depot according to one of the Claims 1 to 4 for the production of a drug releasing anti-angiogenetic substances and genetic materials influencing these systems.

16. Use of an implantable active agent depot according to one of the Claims 1 to 4 for the production of a drug releasing anti-angiogenetic substances and materials influencing these systems.

## Revendications

1. Dépôt de principe actif implantable se compose de
- une matrice lipidique à partir de mono-oléine de glycérine qui est en mesure de former des phases cubiques, dans laquelle
- des molécules lipidiques sont intégrées avec des groupes de tête chargés et/ou à encombrement stérique en tant que molécules modificatrices, ces molécules modificatrices portant le groupe de tête chargé négativement d'acide 1,2-dimyristoyl-glycérophosphatidyle (DMPA, sel de sodium) ou étant employées en tant que molécules modificatrices amphiphiles avec des groupes de tête PEG et qui contient
- des substances à effet pharmaceutique Carboplatine, Oxaliplatine, Taxol, Daunorubicine, Mitoxantrone, Gencitabine, Topotecan, Campothecine, des peptides, des préparations de thérapie génique telles que l'ADN, l'ARN, les oligonucléotides ou des ribozymes.

2. Dépôt de principe actif implantable selon la revendication 1, les molécules modificatrices étant des molécules amphiphiles avec des groupes de tête PEG de longueurs différents, de préférence entre 500 et 2000 unités.

3. Dépôt de principe actif implantable selon la revendication 1 et 2, les molécules modificatrices étant des 1,2-distearoyl-glycérophosphastidyl-éthanolamine-méthyle-poléthylèneglycol (MPEG-DSPE).

4. Dépôt de principe actif implantable selon la revendication 1, plusieurs substances à effet pharmaceutique telles que la combinaison de Carboplatine et de Taxol étant employées.

5. Procédé de fabrication d'un dépôt de principe actif implantable selon l'une des revendications 1 à 4, **se caractérisant par le fait que** l'on ajoute ensemble de préférence à la matrice lipidique, des mono-oléines, des molécules modificatrices et des substances à effet pharmaceutique liposolubles.

6. Procédé de fabrication d'un dépôt de principe actif implantable selon l'une des revendications 1 à 4, **se caractérisant par le fait que** l'on ajoute de préférence à la matrice lipidique, des mono-oléines, des molécules modificatrices et que l'on mélange ensuite avec une phase aqueuse celle qui contient les substances à effet pharmaceutique hydrosolubles.

7. Procédé de fabrication d'un dépôt de principe actif implantable selon l'une des revendications 1 à 4, **se caractérisant par le fait que** l'on ajoute ensemble de préférence à la matrice lipidique, des mono-oléines, des molécules modificatrices et des substances à effet pharmaceutique liposolubles et que l'on mélange ensuite avec une phase aqueuse celle qui contient les substances à effet pharmaceutique hydrosolubles.

8. Emploi d'un dépôt de principe actif implantable selon l'une des revendications 1 à 4 pour la fabrication d'un médicament pour la chimiothérapie locale voire la thérapie génétique de maladies tumorales (entre autres des glioblastomes, des métastases dans le cerveau, des cancer du péritonéaux, des cancers de la vessie, des récidives mammaires).

9. Emploi d'un dépôt de principe actif implantable selon l'une des revendications 1 à 4 pour la fabrication d'un médicament pour le traitement des parois vasculaires artérioscléreuses (entre autres resténoses).

10. Emploi d'un dépôt de principe actif implantable selon l'une des revendications 1 à 4 pour la fabrication d'un médicament pour le traitement de la maladie de Parkinson, d'Alzheimer et de la sclérose en plaques.

11. Emploi d'un dépôt de principe actif implantable selon l'une des revendications 1 à 4 pour la fabrication d'un médicament qui sera employé en tant que système slow release pour les médicaments de traitement de la douleur (entre autres la morphine).

12. Emploi d'un dépôt de principe actif implantable selon l'une des revendications 1 à 4 pour la fabrication d'un médicament pour le traitement de maladies rhumatismales (entre autre arthrite rhumatismale).

13. Emploi d'un dépôt de principe actif implantable selon l'une des revendications 1 à 4 pour la fabrication d'un médicament pour la libération de substances anti-inflammatoires.

14. Emploi d'un dépôt de principe actif implantable selon l'une des revendications 1 à 4 pour la fabrication d'un médicament, **se caractérisant par le fait que** le dépôt de principe actif est appliqué sur des réseaux biodégradables.

15. Emploi d'un dépôt de principe actif implantable selon l'une des revendications 1 à 4 pour la fabrication d'un médicament qui libère des substances antiangiogénétiques et du matériel génétique influençant ces systèmes.

16. Emploi d'un dépôt de principe actif implantable selon l'une des revendications 1 à 4 pour la fabrication d'un médicament qui libère des substances angiogénétiques et du matériel influençant ces systèmes.
